# FASCICULE DE BREVET EUROPEEN

(11) **EP 0 424 231 B1**
(45) Date de publication et mention de la délivrance du brevet: **08.06.1994**
(21) Numéro de dépôt: 90402877.6
(22) Date de dépôt: 16.10.1990
(51) Int. Cl.: A61B 17/22, B06B 3/00

(54) **Dispositif de percussion à ultrasons**
Ultraschallperkussionsgerät
Ultrasonic percussion device

(30) Priorité: 18.10.1989 FR 8913618
(43) Date de publication de la demande: 24.04.1991
(73) Titulaire: AEROSPATIALE Société Nationale Industrielle, 75781 Paris Cédex 16 (FR)
(72) Inventeur: Bocquet, Michel, F-92130 Issy-Les-Moulineaux (FR); Drobinski, Gérard, F-92260 Fontenay aux Roses (FR); Kremer, Daniel, F-77380 Combes La Ville (FR)
(74) Mandataire: Bonnetat, Christian

(56) Documents cités:
- WO-A-89/06515
- US-A- 2 071 954
- US-A- 3 990 452
- US-A- 4 854 325

## Description

La présente invention concerne un dispositif de percussion à ultrasons.

Bien qu'un tel dispositif de percussion puisse être utilisé pour de nombreuses applications, telles que par exemple usinage, polissage, débouchage de conduits, etc..., il sera plus particulièrement décrit ci-après en rapport avec son usage médical. Il va de soi qu'il n'en résulte pas pour autant une limitation de la portée de la présente invention au domaine médical. Dans son application médicale, le dispositif selon l'invention est destiné au traitement des canaux corporels ou conduits morphologiques, notamment à la destruction d'athéromes à l'intérieur des artères.

Pour détruire des athéromes, les praticiens peuvent utiliser, outre des dispositifs à laser ou à outil rotatif coupant, des dispositifs à ultrasons. Ces derniers comprennent, principalement, un générateur à ultrasons auquel est reliée, par l'intermédiaire de moyens de liaison, l'extrémité proximale d'un fil, tandis que l'extrémité distale dudit fil est introduite par le praticien dans le canal corporel à traiter jusqu'à venir au contact des athéromes à briser. La destruction de ceux-ci s'effectue grâce aux vibrations engendrées par le générateur et transmises au fil, dont l'extrémité distale martèle cycliquement les athéromes. Un tel dispositif est décrit dans WO-A- 89 06 515.

Dans la réalité, ces dispositifs à ultrasons, bien qu'étant basés sur un principe largement éprouvé et techniquement maîtrisé et bien que donnant des résultats encourageants, n'en présentent pas moins des inconvénients, notamment au niveau de la liaison entre l'extrémité proximale du fil et le générateur à ultrasons.

En effet, la fixation de l'extrémité proximale du fil au générateur à ultrasons doit être telle que les vibrations engendrées par le générateur soient transmises intégralement au fil pour obtenir un résultat optimal. Or, il s'avère que les moyens de liaison employés actuellement, à cause notamment du diamètre millimétrique du fil et des jeux fonctionnels inévitables qui se produisent lors des vibrations, ne permettent pas d'assurer une transmission maximale des vibrations ultrasonores.

Par suite, l'énergie des vibrations du fil est réduite et ne permet pas une destruction rapide et efficace des athéromes. Pour pallier cette perte d'énergie, le praticien a tendance à augmenter l'énergie des vibrations délivrées par le générateur, mais alors, l'énergie calorifique produite et dégagée au niveau de la liaison fil-générateur est telle qu'elle provoque bien souvent la rupture du fil.

Par ailleurs, la liaison entre l'extrémité proximale du fil et le générateur peut être réalisée au moyen d'une soudure. Cependant, dans ce cas, les contraintes mécaniques et thermiques produites rendent le fil très cassant, entraînant fréquemment sa rupture. En outre, une telle réalisation des moyens de liaison n'autorise plus le démontage du fil.

La présente invention a pour objet de remédier à ces inconvénients et concerne un dispositif de percussion à ultrasons destiné par exemple à la destruction des athéromes dans les artères, dans lequel la conception des moyens de liaison fil-générateur est telle qu'elle permet de transmettre en totalité et en toute sécurité les vibrations engendrées par le générateur audit fil, tout en autorisant de plus le démontage du fil.

A cet effet, le dispositif de percussion à ultrasons, comportant un générateur à ultrasons et un fil, dont l'extrémité proximale est reliée audit générateur par l'intermédiaire de moyens de liaison, de sorte que l'extrémité distale dudit fil est animée d'un mouvement de percussion, est remarquable, selon l'invention, en ce que lesdits moyens de liaison comprennent :
- un manchon radialement déformable de façon élastique, recevant l'extrémité proximale dudit fil et pourvu d'une partie évasée de forme conique ;
- un perçage prévu dans un organe solidaire dudit générateur et dans lequel est susceptible d'être introduit ledit manchon, la partie conique dudit manchon venant au contact d'une paroi conique complémentaire ménagée dans ledit perçage ; et
- un élément de serrage susceptible de coopérer par vissage avec ledit organe et d'agir contre ledit manchon, pour que, lors du vissage, la partie conique dudit manchon soit pressée contre la paroi conique complémentaire dudit perçage en entraînant, par la déformation radiale dudit manchon, le serrage de l'extrémité proximale dudit fil, jusqu'à ce que ledit élément de serrage vienne au contact d'une butée prévue sur ledit organe.

Ainsi, selon l'invention, les vibrations ultrasonores, engendrées par le générateur à ultrasons, sont transmises audit fil de façon optimale, grâce à la liaison conique entre l'organe solidaire du générateur et le manchon serrant l'extrémité proximale du fil, ladite liaison conique étant obtenue grâce à l'élément de serrage qui presse, en la déformant radialement, la partie conique du manchon contre celle correspondante dudit perçage et qui vient en butée contre ledit organe, en créant de plus une liaison additionnelle.

Par ailleurs, on remarquera que le fil peut être aisément démontable par simple dévissage de l'élément de serrage, le manchon, radialement déformable de façon élastique, reprenant sa position initiale. Lorsque le praticien a procédé à la destruction d'un dépôt contenu dans un canal corporel au moyen des vibrations ultrasonores transmises par le fil, il peut ensuite retirer ce dernier du canal corporel et introduire dans ce dernier, par exemple, un cathéter dont l'extrémité distale comporte un ballonnet déformable. De la sorte, le praticien peut procéder au traitement de la portion dudit canal qui contenait préalablement le dépôt par déformation de celle-ci, un tel cathéter à ballonnet ne pouvant être utilisé antérieurement puisque la portion à traiter dudit canal était obstruée par le dépôt à détruire.

Dans une réalisation préférée, ledit manchon comporte une partie cylindrique circulaire prolongée par ladite partie évasée conique, ladite partie cylindrique étant susceptible de coopérer avec la paroi dudit perçage ménagé dans ledit organe, tandis que la partie évasée conique dudit manchon vient en appui contre la paroi conique complémentaire dudit perçage.

De la sorte, la partie cylindrique assure le positionnement correct du manchon par rapport à l'organe solidaire du générateur à ultrasons, tandis que la partie évasée conique permet le serrage de l'extrémité proximale du fil.

Pour autoriser la déformation radiale de façon élastique dudit manchon, des fentes radiales sont ménagées dans au moins la partie évasée conique dudit manchon. Avantageusement, quatre fentes radiales sont prévues dans ledit manchon en étant équi-angulairement réparties les unes des autres. Ainsi, la partie évasée conique dudit manchon est formée approximativement par quatre quarts de cône, dont les arêtes internes, parallèles à la direction longitudinale dudit manchon, viennent s'appuyer contre l'extrémité proximale du fil, en la serrant fermement.

Par ailleurs, ledit élément de serrage peut comprendre une paroi latérale cylindrique taraudée, terminée par un fond ouvert venant au contact de la face d'extrémité de la partie évasée dudit manchon en autorisant le passage dudit fil, tandis que la face d'extrémité de ladite paroi latérale cylindrique dudit élément de serrage, opposée audit fond ouvert, vient en appui contre la butée dudit organe.

Selon une autre caractéristique de l'invention, le diamètre du fil est compris entre 0,15 mm et 0,50 mm. Avantageusement, le diamètre du fil est sensiblement égal à 0,4 mm. Ainsi, le serrage de l'extrémité proximale du fil s'effectue dans des conditions satisfaisantes, pour permettre aux arêtes internes des quatre quarts de cône, formant notamment la partie évasée conique du manchon, de pincer fermement ladite extrémité proximale du fil.

Dans une forme préférée de réalisation, l'extrémité distale dudit fil est amincie. Avantageusement, l'extrémité distale dudit fil présente une section oblongue, dont l'une des dimensions correspond au diamètre du fil tandis que l'autre dimension est inférieure audit diamètre. De la sorte, l'extrémité distale du fil présente une certaine flexibilité dans la direction qui correspond à celle de dimension inférieure. Le praticien peut alors diriger de façon appropriée l'extrémité distale du fil, pour faciliter la progression du fil dans les sinuosités du canal corporel.

De plus, pour permettre au praticien de suivre, sur un écran de visualisation, le cheminement du fil dans le canal corporel à traiter et contrôler l'efficacité du traitement par ultrasons qui s'ensuit, le fil est avantageusement réalisé en une matière radio-opaque.

Les figures du dessin annexé feront bien comprendre comment l'invention peut être réalisée. Sur ces figures, des références identiques désignent des éléments semblables.

La figure 1 représente schématiquement, en perspective, un exemple de réalisation du dispositif de percussion à ultrasons selon l'invention.

La figure 2 est une vue éclatée en coupe des moyens de liaison avant assemblage, destinés à relier l'extrémité proximale du fil au générateur à ultrasons.

La figure 3 est une vue du manchon radialement déformable selon la flèche F de la figure 2.

La figure 4 est une vue en coupe des moyens de liaison après assemblage reliant l'extrémité proximale du fil au générateur à ultrasons.

La figure 5 montre en perspective un mode préféré de réalisation de l'extrémité distale dudit fil.

La figure 6 est une vue en coupe schématique d'un canal corporel, tel qu'une artère, obstrué par un athérome à détruire contre lequel agit l'extrémité distale du fil.

Les figures 7a et 7b représentent schématiquement le mode d'actionnement de l'extrémité distale du fil contre l'athérome à détruire.

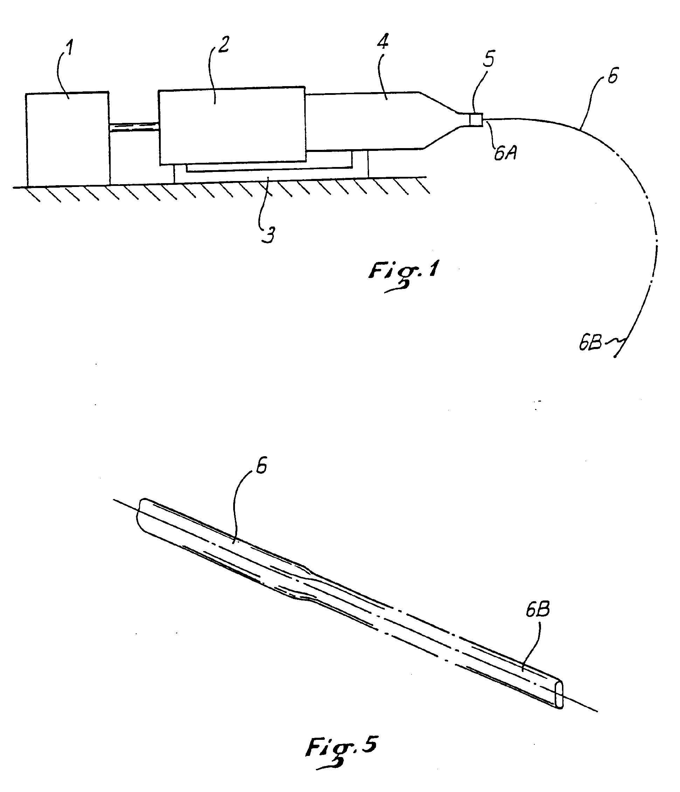

En se référant à la figure 1, le dispositif de percussion à ultrasons comporte un générateur d'ondes ultrasonores 1, d'un type connu en soi, auquel est relié un transducteur piézo-électrique 2 monté sur un support 3. Le transducteur 2 reçoit, par l'intermédiaire de moyens de transmission 4 pourvus de moyens de liaison 5, l'extrémité proximale 6A d'un fil 6, dont l'extrémité distale 6B est susceptible d'être introduite dans un canal corporel à traiter pour venir au contact d'obstacles, tels que des dépôts entraînant la formation d'athéromes à l'intérieur des artères, afin de les détruire. La destruction de la formation athéromateuse est obtenue à partir du générateur à ultrasons et du transducteur 2, lequel convertit l'énergie électrique produite par le générateur à ultrasons en énergie mécanique transmise sous la forme de vibrations mécaniques le long du fil, l'extrémité distale du fil martelant alors cycliquement la formation obstructive athéromateuse.

Pour que l'énergie mécanique engendrée par le transducteur soit transmise intégralement au fil, les moyens de liaison 5, reliant l'extrémité proximale 6A du fil au transducteur 2, comprennent, selon l'invention, un manchon 7 radialement déformable de façon élastique, un perçage 8 prévu dans un organe 9 de révolution solidaire des moyens de transmission 4, et un élément de serrage 10.

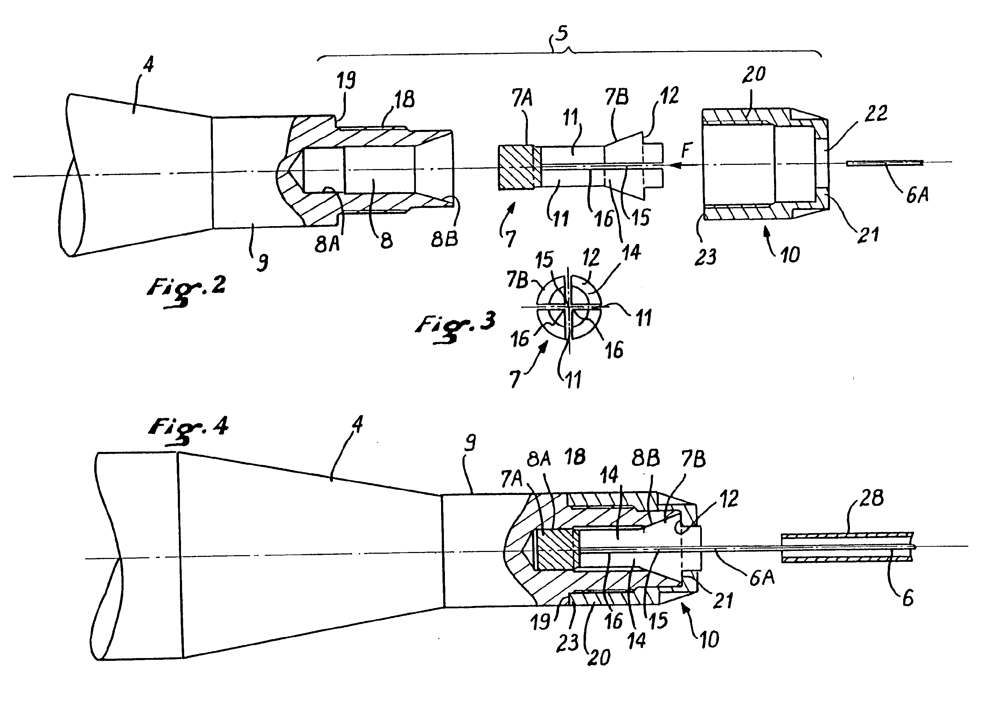

En se référant à la figure 2, qui montre les moyens de liaison 5 avant leur assemblage, le manchon 7, destiné à recevoir l'extrémité proximale 6A du fil, comporte principalement une partie cylindrique circulaire 7A prolongée par une partie évasée de forme conique 7B. En outre, des fentes radiales 11 sont ménagées dans le manchon 7 et s'étendent, dans ce mode de réalisation, depuis la face d'extrémité 12 de la partie évasée conique 7B jusque dans la partie circulaire 7A.

Sur la figure 3, on voit notamment que quatre fentes radiales 11 sont prévues dans le manchon en étant équi-angulairement réparties les unes des autres et en délimitant, à l'endroit où ces fentes sont pratiquées, quatre portions 14 identiques approximativement tronconiques. Deux fentes opposées se trouvent par conséquent dans le prolongement l'une de l'autre dans un plan diamétral commun longitudinal audit manchon. Grâce à ces fentes 11, le manchon 7 peut être radialement déformable, de façon élastique.

L'extrémité proximale 6A du fil est susceptible d'être introduite dans le passage axial 15 délimité par les arêtes internes 16 des quatre portions 14, pour y être fermement maintenue par celles-ci.

Sur la figure 2, le perçage 8 pratiqué dans l'organe 9 solidaire du transducteur 2 présente, à son extrémité débouchante, une paroi conique 8B prolongeant la paroi cylindrique 8A dudit perçage. Ainsi, la partie cylindrique 7A du manchon est susceptible de coopérer avec la paroi cylindrique 8A du perçage 8, tandis que la partie évasée 7B du manchon est susceptible de coopérer avec la paroi conique 8B, de forme complémentaire, dudit perçage.

Par ailleurs, il est également prévu un filetage 18 sur l'organe 9, concentrique au perçage 8 et terminé par un épaulement annulaire radial 19 formant butée.

L'élément de serrage 10 comprend une paroi latérale cylindrique taraudée 20, destinée à coopérer avec le filetage 18 ménagé sur l'organe 9 et terminée par un fond 21 pourvu d'une ouverture 22, permettant le passage de l'extrémité proximale 6A du fil 6.

La face d'extrémité 23 de la paroi latérale 20 de l'élément de serrage, opposée au fond 22, est destinée à venir en butée contre l'épaulement annulaire 19 prévu sur l'organe 9.

L'assemblage des moyens de liaison 5 permettant le serrage de l'extrémité proximale 6A du fil 6 et la transmission des vibrations engendrées est montré sur la figure 4.

On y voit que le manchon 7 est introduit dans le perçage 8 de l'organe 9, solidaire du transducteur 2, de façon que sa partie cylindrique 7A coopère, par un ajustement, avec la paroi cylindrique correspondante 8A du perçage 8, ce qui assure de la sorte un positionnement et un centrage appropriés du manchon 7 par rapport au perçage 8 de l'organe 9.

Le manchon 7 est introduit jusqu'à ce que sa partie évasée conique 7B vienne au contact de la paroi conique 8B dudit perçage.

L'extrémité proximale 6A est engagée dans le passage axial 15 du manchon jusqu'à venir en butée contre le fond des fentes radiales 11, les arêtes internes 16 des portions 14 affleurant l'extrémité proximale 6A du fil.

L'élément de serrage 10, par le fond 21 duquel passe le fil 6, est alors vissé par sa paroi latérale taraudée 20 sur le filetage 18 ménagé sur l'organe 9.

Lors du vissage, le fond ouvert 21 de l'élément 10 vient en appui contre la face d'extrémité 12 de la partie conique 7B du manchon en pressant ainsi ladite partie conique contre la paroi conique 8B complémentaire du perçage 8. Cela entraîne, grâce aux fentes radiales 11, la déformation radiale des portions 14 du manchon 7 qui s'appliquent fortement contre la paroi conique 8B, et, simultanément, le serrage de l'extrémité proximale 6A du fil par les arêtes internes 16 desdites portions. L'élément de serrage 10 est vissé jusqu'à venir en butée, par sa face d'extrémité 23, contre l'épaulement annulaire radial 19 de l'organe 9.

On conçoit donc qu'avec ces moyens de liaison 5, les vibrations engendrées par le générateur à ultrasons et reproduites par l'organe 9 lié au transducteur 2 sont transmises de façon optimale au fil 6.

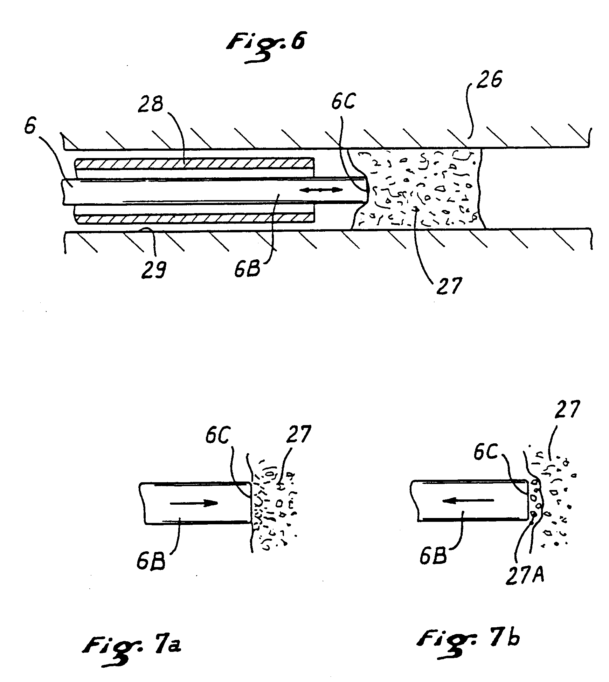

Par ailleurs, l'extrémité distale 6B de ce dernier est amincie, comme le montre la figure 5. Cette extrémité 6B présente avantageusement une section oblongue dont l'une des dimensions correspond au diamètre du fil, tandis que l'autre dimension, perpendiculaire à la précédente, est bien inférieure au diamètre du fil. Grâce à la flexibilité de l'extrémité distale 6B du fil dans la direction de petite dimension, le praticien peut orienter au mieux l'extrémité distale du fil pour suivre les sinuosités du canal corporel à traiter.

Le fil est avantageusement réalisé en une matière radio-opaque permettant au praticien de suivre la position du fil dans le canal corporel, et l'efficacité du traitement proprement dit. Le diamètre du fil peut être d'environ 0,4 millimètre et sa longueur peut être comprise entre 1 et 2,5 mètres en fonction de la longueur du canal corporel à traiter et de l'emplacement de la formation athéromateuse. L'extrémité distale 6B du fil est avantageusement amincie sur une longueur comprise entre 0,6 et 0,8 mètre.

Par ailleurs, pour éviter les fréquences parasites transversalement audit fil, la fréquence des vibrations émise par le générateur à ultrasons coïncide avec la fréquence propre de résonance du fil.

On a représenté schématiquement sur la figure 6 une partie d'une artère 26 obstruée, par exemple, par une formation athéromateuse 27.

Pour procéder à l'élimination de cette formation, le praticien introduit préalablement une gaine 28 dans l'artère 26 jusqu'au voisinage de la formation athéromateuse 27. Cette gaine 28 assure le guidage du fil 6 dans l'artère et protège la paroi interne 29 de l'artère 26 des frottements du fil qui pourraient occasionner des échauffements et des brûlures néfastes de l'artère.

Lorsque la face 6C de l'extrémité distale 6B du fil est sensiblement au contact de la formation athéromateuse 27, le dispositif à ultrasons 1 selon l'invention est mis en action, les vibrations engendrées étant transmises de façon optimale au fil grâce aux moyens de liaison préalablement décrits.

Les vibrations du fil, transmises longitudinalement, agissent contre la formation athéromateuse de la façon illustrée sur les figures 7a et 7b. Lors du mouvement d'avance du fil, la face 6C de l'extrémité distale 6B heurte la formation 27 pour provoquer progressivement l'effrittement et la fragmentation de celle-ci. En revanche, lors du mouvement de recul du fil, les particules 27A de la formation athéromateuse sont enlevées progressivement de l'athérome par des effets de dépression ou de cavitation.

Par ailleurs, le praticien peut ensuite retirer le fil de l'artère pour y introduire un cathéter dont l'extrémité distale est pourvue d'un ballonnet déformable. De la sorte, en positionnant le ballonnet dans l'artère à l'endroit de la formation athéromateuse alors détruite, le praticien peut procéder, de façon classique, à une dilatation de l'artère.

## Revendications

1. Dispositif de percussion à ultrasons comportant un générateur à ultrasons (1,2) et un fil (6) dont l'extrémité proximale (6A) est reliée audit générateur par l'intermédiaire de moyens de liaison (5), de sorte que l'extrémité distale (6B) dudit fil est animée d'un mouvement de percussion,
caractérisé en ce que lesdits moyens de liaison (5) comprennent :
- un manchon (7) radialement déformable de façon élastique, recevant l'extrémité proximale dudit fil et pourvu d'une partie évasée (7B) de forme conique ;
- un perçage (8) prévu dans un organe (9) solidaire dudit générateur et dans lequel est susceptible d'être introduit ledit manchon, la partie conique (7B) dudit manchon (7) venant au contact d'une paroi conique complémentaire (8B) ménagée dans ledit perçage ; et
- un élément de serrage (10) susceptible de coopérer par vissage avec ledit organe (9) et d'agir contre ledit manchon (7) pour que, lors du vissage, la partie conique (7B) dudit manchon soit pressée contre la paroi conique complémentaire (8B) dudit perçage en entraînant, par la déformation radiale dudit manchon, le serrage de l'extrémité proximale (6A) dudit fil, jusqu'à ce que ledit élément de serrage (10) vienne au contact d'une butée (19) prévue sur ledit organe.

2. Dispositif selon la revendication 1,
caractérisé en ce que ledit manchon (7) comporte une partie cylindrique circulaire (7A) prolongée par ladite partie évasée conique (7B), ladite partie cylindrique (7A) étant susceptible de coopérer avec la paroi (8A) dudit perçage ménagé dans ledit organe, tandis que la partie évasée conique (7B) dudit manchon vient en appui contre la paroi conique complémentaire (8B) dudit perçage.

3. Dispositif selon l'une des revendications 1 ou 2, caractérisé en ce que des fentes radiales (11) sont ménagées dans au moins la partie évasée conique dudit manchon (7), autorisant la déformation radiale de celui-ci.

4. Dispositif selon la revendication 3,
caractérisé en ce que quatre fentes radiales (11) sont prévues dans ledit manchon (7) en étant équi-angulairement réparties les unes des autres.

5. Dispositif selon l'une quelconque des revendications précédentes 1 à 4,
caractérisé en ce que ledit élément de serrage (10) comprend une paroi latérale cylindrique taraudée (20), terminée par un fond ouvert (21) venant au contact de la face d'extrémité (12) de la partie évasée (7B) dudit manchon en autorisant le passage dudit fil, tandis que la face d'extrémité (23) de ladite paroi latérale cylindrique (20) dudit élément de serrage, opposée audit fond ouvert, vient en appui contre la butée (19) dudit organe.

6. Dispositif selon l'une des revendications précédentes 1 à 5,
caractérisé en ce que le diamètre dudit fil (6) est compris entre 0,15 mm et 0,50 mm.

7. Dispositif selon la revendication 6,
caractérisé en ce que le diamètre dudit fil est sensiblement égal à 0,4 mm.

8. Dispositif selon l'une quelconque des revendications précédentes 1 à 7,
caractérisé en ce que l'extrémité distale (6B) dudit fil (6) est amincie.

9. Dispositif selon la revendication 8,
caractérisé en ce que l'extrémité distale (6B) dudit fil présente une section oblongue, dont l'une des dimensions correspond au diamètre dudit fil, tandis que l'autre dimension est inférieure au diamètre dudit fil.

10. Dispositif selon l'une quelconque des revendications 1 à 9,
caractérisé en ce que ledit fil (6) est réalisé en une matière radio-opaque.

## Patentansprüche

1. Ultraschallschlagvorrichtung mit einem Ultraschallgenerator (1,2) und einem Draht (6), dessen proximales Ende (6A) mit dem Generator durch Verbindungsmittel (5) verbunden ist, so daß das distale Ende (6B) des Drahtes eine Schlagbewegung ausführt,
dadurch gekennzeichnet, daß die Verbindungsmittel (5) umfassen:
- eine radial elastisch verformbare Muffe (7), die das proximale Ende des Drahtes aufnimmt, mit einem aufgeweiteten konischen Teil (7B);
- eine Bohrung (8) in einem mit dem Generator fest verbundenen Organ (9), in die die Muffe eingeführt werden kann, wobei der konische Teil (7B) der Muffe (7) mit einer entsprechenden konischen Wand (8B) in der Bohrung in Berührung kommt; und
- ein Spannelement (10), das durch Aufschrauben mit dem Organ (9) zusammenwirken und auf die Muffe (7) einwirken kann, so daß der konische Teil (7B) der Muffe beim Aufschrauben gegen die entsprechende konische Wand (8B) der Bohrung gedrückt wird und durch die radiale Verformung der Muffe zum Klemmen des proximalen Endes (6A) des Drahtes führt, bis das Spannelement (10) an einem Anschlag (19) am Organ anliegt.

2. Vorrichtung nach Anspruch 1,
dadurch gekennzeichnet, daß die Muffe (7) einen kreisrunden zylindrischen Teil (7A) hat, an den sich der aufgeweitete konische Teil (7B) anschließt, wobei der zylindrische Teil (7A) mit der Wand (8A) der im Organ vorgesehenen Bohrung zusammenwirken kann, während der aufgeweitete konische Teil (7B) der Muffe an der entsprechenden konischen Wand (8B) der Bohrung anliegt.

3. Vorrichtung nach einem der Ansprüche 1 oder 2,
dadurch gekennzeichnet, daß mindestens im aufgeweiteten konischen Teil der Muffe (7) Radialspalte (11) vorgesehen sind, die die radiale Verformung der Muffe ermöglichen.

4. Vorrichtung nach Anspruch 3,
dadurch gekennzeichnet, daß in der Muffe (7) vier Radialspalte (11) vorgesehen sind, die gleichwinklig zueinander verteilt sind.

5. Vorrichtung nach einem der obigen Ansprüche 1 bis 4,
dadurch gekennzeichnet, daß das Spannelement (10) eine zylindrische Seitenwand mit Innengewinde (20) hat, die in einem offenen Boden (21) endet, der mit der Endenseite (12) des aufgeweiteten Teils (7B) der Muffe in Berührung kommt und die Durchführung des Drahtes ermöglicht, während die dem offenen Boden gegenüberliegende Endenseite (23) der zylindrischen Seitenwand (20) des Spannelements am Anschlag (19) des Organs anliegt.

6. Vorrichtung nach einem der obigen Ansprüche 1 bis 5,
dadurch gekennzeichnet, daß der Durchmesser des Drahtes (6) zwischen 0,15 mm und 0,50 mm liegt.

7. Vorrichtung nach Anspruch 6,
dadurch gekennzeichnet, daß der Durchmesser etwa gleich 0,4 mm ist.

8. Vorrichtung nach einem der obigen Ansprüche 1 bis 7,
dadurch gekennzeichnet, daß das distale Ende (6B) des Drahtes (6) dünner ist.

9. Vorrichtung nach Anspruch 8,
dadurch gekennzeichnet, daß das distale Ende (6B) des Drahtes einen länglichen Querschnitt hat, dessen eine Abmessung dem Durchmesser des Drahtes entspricht, während die andere Abmessung kleiner als der Durchmesser des Drahtes ist.

10. Vorrichtung nach einem der Ansprüche 1 bis 9,
dadurch gekennzeichnet, daß der Draht (6) aus einem radiopaken Material besteht.

## Claims

1. Ultrasonic percussion device including an ultrasound generator (1, 2) and a wire (6) whose proximal end (6A) is joined to the said generator using linkage means (5) so that the distal end (6) of the said wire is driven in a percussive movement,
characterized in that the said linkage means (5) comprise:
- a sleeve (7) which can be radially deformed in an elastic manner, receiving the proximal end of the said wire and provided with a flared part (7B) of conical shape;
- a hole (8) provided in a member (9) which is integral with the said generator, into which hole the said sleeve can be inserted, the conical part (7B) of the said sleeve (7) coming into contact with a matching conical wall (8B) made in the said hole; and
- a clamping element (10) which can interact, by screwing, with the said member (9) and act against the said sleeve (7) so that, during the screwing, the conical part (7B) of the said sleeve is pressed against the matching conical wall (8B) of the said hole while causing, by radial deformation of the said sleeve, clamping of the proximal end (6A) of the said wire until the said clamping element (10) comes into contact with a stop (19) provided on the said member.

2. Device according to Claim 1, characterized in that the said sleeve (7) includes a circular cylindrical part (7A) extended by the said conical flared part (7B), the said cylindrical part (7A) being capable of interacting with the wall (8A) of the said hole made in the said member, while the conical flared part (7B) of the said sleeve comes to bear against the matching conical wall (8B) of the said hole.

3. Device according to one of Claims 1 or 2, characterized in that radial slits (11) are made in at least the conical flared part of the said sleeve (7), allowing the radial deformation of the latter.

4. Device according to Claim 3, characterized in that four radial slits (11) are provided in the said sleeve (7), while being distributed at equal angles from each other.

5. Device according to any one of the preceding Claims 1 to 4, characterized in that the said clamping element (10) comprises a tapped cylindrical side wall (20), terminated by an open base (21) which comes into contact with the end face (12) of the flared part (7B) of the said sleeve, leaving clearance for the said wire, while the end face (23) of the said cylindrical side wall (20) of the said clamping element which face is opposite the said open base, comes to bear against the stop (19) of the said member.

6. Device according to any one of the preceding Claims 1 to 5, characterized in that the diameter of the said wire (6) is between 0.15 mm and 0.50 mm.

7. Device according to Claim 6, characterized in that the diameter of the said wire is substantially equal to 0.4 mm.

8. Device according to any one of the preceding Claims 1 to 7, characterized in that the distal end (6B) of the said wire (6) is thinned.

9. Device according to Claim 8, characterized in that the distal end (6B) of the said wire has an oblong cross-section, one of whose dimensions corresponds to the diameter of the said wire, while the other dimension is less than the diameter of the said wire.

10. Device according to any one of Claims 1 to 9, characterized in that the said wire (6) is made of a radiopaque material.
